# FASCICULE DE BREVET EUROPEEN

(11) **EP 0 701 830 B1**
(45) Date de publication et mention de la délivrance du brevet: **20.06.2001**
(21) Numéro de dépôt: 95420250.3
(22) Date de dépôt: 05.09.1995
(51) Int. Cl.: A61M 1/36, G01L 11/00

(54) **Dispositif de mesure de la pression d'un liquide et procédé de réglage d'un tel dispositif**
Flüssigkeitsdruckmessvorrichtung und Regelungsverfahren einer solchen Vorrichtung
Device for measuring the pressure of a liquid and procedure for regulating such a device

(30) Priorité: 14.09.1994 FR 9411216
(43) Date de publication de la demande: 20.03.1996
(73) Titulaire: HOSPAL INDUSTRIE, F-69883 Meyzieu Cédex (FR)
(72) Inventeur: Chevallet, Jacques, F-69360 Serezin du Rhone (FR); Frugier, Alain, F-38230 Tignieu (FR); Louvet, Eric, F-38090 Villefontaine (FR)
(74) Mandataire: Lejeune, Daniel

(56) Documents cités:
- EP-A- 0 611 227
- EP-A- 0 611 228
- FR-A- 2 655 419
- US-A- 3 635 089
- US-A- 3 863 504
- US-A- 4 546 785

## Description

La présente invention concerne un dispositif de mesure de la pression d'un liquide et un procédé de réglage d'un tel dispositif.

Le dispositif de mesure de pression auquel a trait l'invention est du type comprenant un capteur de pression sensible à la pression d'un gaz et un boîtier divisé en deux compartiments par une membrane souple non perméable, un premier compartiment ayant au moins un orifice pour y introduire le liquide et le second compartiment étant connectable, par des moyens de raccord étanches, au capteur de pression. Les variations de pression dans le liquide sont transmises par la membrane au gaz présent dans le second compartiment et les variations de pression du gaz sont mesurées par le capteur.

La demande de brevet français 2 655 419 décrit un dispositif de mesure de pression comprenant un boîtier tubulaire divisé en deux compartiments par une chambre à vide tubulaire, déplaçable axialement dans le boîtier, de part et d'autre d'un point d'ancrage fixe. La paroi tubulaire de la chambre à vide est constitué par deux soufflets cylindriques s'étendant de part et d'autre du point d'ancrage. Le premier compartiment du boîtier tubulaire est muni d'un orifice de communication avec le milieu dont la pression est à mesurer. Le second compartiment (compartiment de mesure) est relié à des moyens pour établir une contre pression. La chambre à vide se déplace à l'intérieur du boîtier sous l'effet de la pression à mesurer et de la contre pression. La position de la chambre à vide dans le boîtier est repérée au moyen d'un contact solidaire de la chambre à vide, mobile entre deux contacts électriques fixes. La pression à mesurer est déduite de la contre pression nécessaire pour positionner la chambre à vide dans une position d'équilibre où le contact mobile est situé entre les deux contacts fixes. Dans ce dispositif, le positionnement de la chambre à vide tubulaire vis-à-vis des deux compartiments a pour fin de mesurer la pression régnant dans le compartiment de mesure. Par ailleurs, le positionnement de cette chambre à vide ne s'effectue pas à partir de la mesure de la pression dans le compartiment de contre pression, mais de la détection du contact établi entre l'organe de contact mobile, et les organes de contact fixes.

Le brevet US 3 863 504 décrit un dispositif de mesure de pression comprenant:
- une jauge de pression munie d'un cadran à aiguille ;
- un boîtier transparent divisé en deux compartiments par un diaphragme, un premier compartiment ayant un orifice d'entrée et un orifice de sortie pour le raccordement du boîtier à un circuit de liquide, et un second compartiment relié par une canalisation à la jauge de pression ;
- une seringue connectable à la canalisation reliant le deuxième compartiment du boîtier à la jauge de pression, pour pouvoir ajuster la position du diaphragme dans le boîtier.

Pour effectuer une mesure avec ce dispositif, on injecte de l'air dans le second compartiment au moyen de la seringue jusqu'à ce que le diaphragme occupe une position déterminée, où il est opérationnel, observable au travers du boîtier. Quand il est dans cette position, les pressions de part et d'autre du diaphragme sont égales et la pression du liquide peut être lue sur le cadran de la jauge de pression.

Ce type de dispositif de mesure de pression est utilisé en particulier sur des appareils de traitement du sang par circulation extracorporelle comprenant un dispositif de traitement proprement dit, tel qu'un dialyseur ou une centrifugeuse, auquel sont reliées diverses canalisations où plusieurs liquides, dont du sang, sont mis en circulation. Dans ce genre d'appareil, il est indispensable, pour des raisons de sécurité, de vérifier la pression des différents liquides circulant dans les canalisations, qui sont généralement à usage unique. L'appareil est muni de plusieurs capteurs non jetables, avec lesquels, en fonctionnement, coopèrent des boîtiers de mesure de pression disposés sur les canalisations.

Un intérêt de ce type de dispositif de mesure pour la mesure de la pression du sang est qu'il permet d'éviter toute interface entre le sang et l'air et, par là, l'apparition de filaments de sang coagulé tels qu'on peut en observer dans les pièges à bulles disposés sur les circuits de circulation extracorporelle de sang après deux ou trois heures d'utilisation.

En contrepartie, il arrive qu'en cours d'utilisation la membrane vienne en butée contre l'une ou l'autre de deux parois opposées du boîtier (selon que le liquide est en surpression ou en dépression par rapport à l'atmosphère), rendant le dispositif inapte à détecter des variations de pression. Une légère fuite de gaz au raccordement entre le second compartiment et le capteur de pression peut être à l'origine de ce dysfonctionnement. On fait ici l'hypothèse que, au moins avec certains types de membrane, il pourrait être causé aussi par la perméation du gaz au travers de la membrane, du premier compartiment vers le second compartiment. Cette mise hors service progressive du dispositif ne peut pas être détectée au moyen d'une simple mesure de pression, car la pression peut rester dans une fourchette de pressions jugée acceptable même lorsque la membrane ne répond plus.

La demande de brevet européen n° 0 611 228 décrit un procédé pour détecter que la membrane est venue en butée sur une paroi du boîtier et que le dispositif de mesure de pression est hors service. Selon cette demande, pour détecter si la membrane est toujours opérationnelle, on provoque à intervalles de temps donnés une variation instantanée du débit du liquide circulant dans le premier compartiment, on calcule la variation de la pression, par rapport au temps, dans le second compartiment, et on compare cette variation à une valeur prédéterminée correspondant à la réponse d'une membrane opérationnelle.

Lorsque la mise hors service du dispositif de mesure de pression a été détectée, le problème qui se pose est de remédier à l'anomalie sans avoir à changer la canalisation sur laquelle est disposé le boîtier défectueux. Ce problème ne semble pas avoir été identifié en tant que tel à ce jour.

Un but de l'invention est donc de réaliser un dispositif de mesure de pression du type mentionné plus haut dont la mesure reste fiable quel que soit le temps d'utilisation.

Pour atteindre ce but, on prévoit, selon l'invention, un dispositif de mesure de la pression d'un liquide comportant :
- un capteur de pression sensible à une variation de pression gazeuse ;
- un boîtier divisé en deux compartiments par une membrane souple ayant deux faces opposées tournées respectivement vers chacuns des deux compartiments, un premier compartiment ayant au moins un orifice pour l'introduction du liquide et un second compartiment étant connectable de façon étanche au capteur de pression ; et
- des moyens de pompage pour faire varier la quantité de gaz dans le deuxième compartiment,
- des moyens de commande pour piloter les moyens de pompage en fonction des variations de pressions induites dans le second compartiment par les moyens de pompage de façon à ajuster la position de la membrane entre une paroi du boîtier délimitant le premier compartiment et une paroi du boîtier délimitant le deuxième compartiment.

Ce dispositif procure deux avantages substantiels, l'un d'ordre thérapeutique, l'autre d'ordre pécuniaire, d'autant plus sensibles qu'il fait partie d'un ensemble machine/module de traitement jetable tel que décrit dans les demandes de brevet européen n° 0 611 228 et n° 0 611 227. On rappelle que le module de traitement jetable qui fait l'objet d'une de ces demandes comprend un appareil de traitement du sang (hémodialyseur, par exemple) auquel sont connectées de multiples canalisations dont certaines comportent des boîtier de mesures de pression à membrane. Grâce au dispositif selon l'invention, ce module hautement intégré peut n'être changé que lorsque l'appareil de traitement de sang nécessite de l'être et sa durée d'utilisation n'est pas limitée par la défaillance possible d'un dispositif de mesure de pression. Ceci est avantageux au plan thérapeutique dans la mesure ou les opérations de déconnexion et de connexion au patient d'un circuit de circulation extracorporelle de sang comporte toujours des risques, de contamination en particulier. Au plan pécuniaire, on comprend immédiatement l'intérêt qu'il y a à pouvoir utiliser les modules de traitement jetables jusqu'à la limite imposée par l'appareil de traitement de sang lui-même.

Un autre intérêt de ce dispositif est qu'il permet de faire l'économie de moyens pour détecter une absence de réaction de la membrane.

Selon une caractéristique de l'invention, les moyens de pompage pour faire varier la quantité de gaz dans le deuxième compartiment du boîtier sont connectables à ce compartiment par une canalisation obturable par une vanne et un second capteur de pression est relié à la portion de canalisation reliant la vanne à la pompe. Des moyens sont prévus pour comparer les informations mesurées par le premier et le second capteur de pression et pour piloter la pompe de façon que les pressions dans la canalisation de part et d'autre de la vanne puisse être rendues sensiblement égales

Grâce à cette disposition, il est possible d'éviter que la membrane soit soumise à des variations de pression brutales lors de chaque phase de réglage.

L'invention a aussi pour objet un appareil de traitement de sang par circulation extracorporelle comportant plusieurs dispositifs de mesure de pression qui sont reliés à une pompe unique par des moyens de connexion permettant un réglage successif de la position des membranes dans les différents boîtiers.

L'invention a aussi pour objet un procédé de réglage d'un dispositif de mesure de pression du type mentionné ci-dessus caractérisé en ce qu'il comporte les étapes de :
- introduire dans le premier compartiment le liquide dont la pression est à mesurer ;
- faire varier la quantité de gaz dans le second compartiment au moins une fois dans un sens et au moins une fois dans le sens opposé jusqu'à ce que le second compartiment contiennent une quantité de gaz correspondant à une position déterminée de la membrane dans le boîtier.

Selon un premier mode de réalisation de l'invention, l'étape de faire varier la quantité de gaz dans le second compartiment comprend les étapes de :
- faire varier dans un sens la quantité de gaz dans le second compartiment jusqu'à ce que la pression du gaz atteigne une valeur de seuil déterminée ;
- régler la position de la membrane dans le boîtier en faisant varier en sens contraire la quantité de gaz dans le second compartiment.

Selon un deuxième mode de réalisation de l'invention l'étape de faire varier la quantité de gaz dans le second compartiment comprend les étapes de :
- faire varier la quantité de gaz dans le second compartiment jusqu'à ce que la pression du gaz dans le second compartiment atteigne une valeur de plateau correspondant à une position de flottement de la membrane, puis évolue dans un seul sens et atteigne une valeur de seuil déterminée ;
- régler la position de la membrane dans le boîtier en faisant varier en sens contraire la quantité de gaz dans le second compartiment.

Selon une caractéristique de l'invention, l'étape de régler la position de la membrane dans le boîtier consiste à introduire ou à extraire du gaz dans/du second compartiment pendant une durée déterminée.

D'autres caractéristiques et avantages de l'invention apparaîtront à la lecture de la description qui suit. On se reportera aux dessins annexés sur lesquels :
La figure 1 est un schéma d'un appareil de traitement de sang comportant plusieurs dispositifs de mesure de pression selon l'invention ;
La figure 2 représente une vue schématique, partiellement en coupe, d'un mode de réalisation du dispositif de l'invention ;
La figure 3 est un diagramme illustrant un procédé de réglage du dispositif selon l'invention ; et
La figure 4 représente un schéma d'un circuit pneumatique reliant plusieurs dispositifs de pressions, destiné à l'appareil de la figure 1.

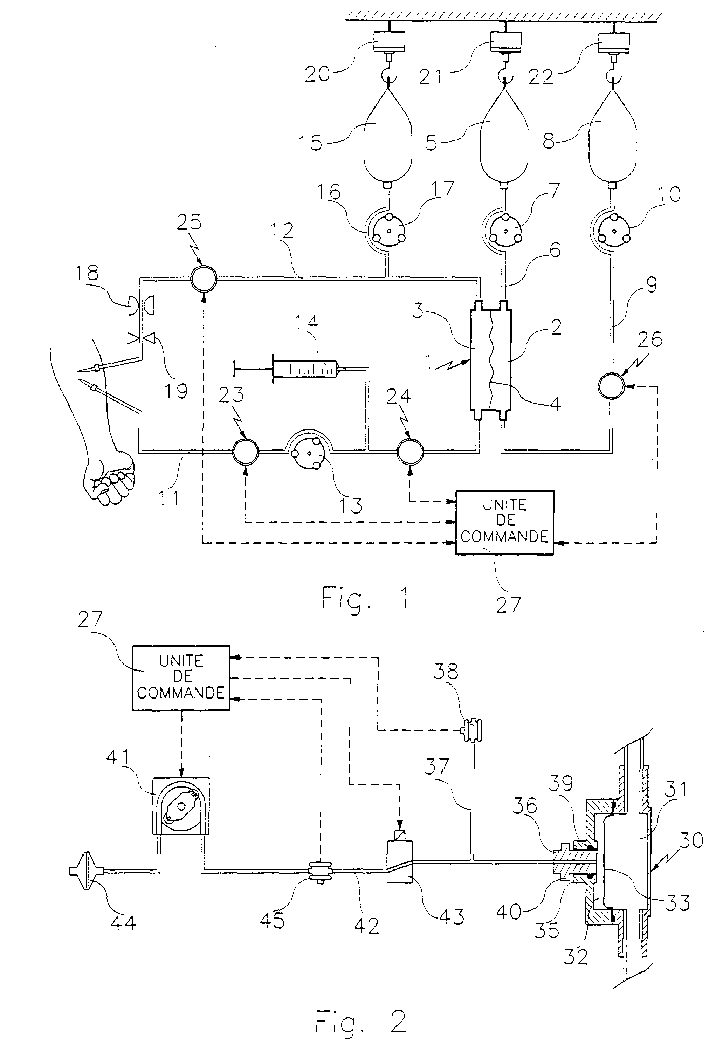

La figure 1 représente un appareil de traitement du sang par circulation extracorporelle grâce auquel plusieurs types de traitement peuvent être administrés, en particulier la dialyse, I'hémofiltration et l'hémodiafiltration. Cet appareil comprend un échangeur 1 à membrane semi-perméable, tel qu'un dialyseur ou un hémofiltre, ayant deux compartiments 2, 3 séparés par une membrane semi-perméable 4. Un premier compartiment 2 a une entrée connectée à une poche de liquide de traitement (liquide de dialyse) 5 par l'intermédiaire d'une canalisation d'alimentation 6 sur laquelle est disposée une pompe d'alimentation 7, et une sortie connectée à une poche de recueil de liquide usé 8 par l'intermédiaire d'une canalisation d'évacuation 9 sur laquelle est disposée une pompe d'extraction 10. Le second compartiment 3 est connecté à un circuit de circulation extracorporelle de sang comprenant une canalisation de prélèvement 11 ayant une canule à une extrémité et étant connectée, à son autre extrémité, à une entrée du second compartiment, et une canalisation de restitution 12 ayant une canule à une extrémité et étant connectée, à son autre extrémité, à une sortie du second compartiment 3. Une pompe de circulation 13 est disposée sur la canalisation de prélèvement 11. Un dispositif d'injection d'anticoagulant 14 est relié à la canalisation de prélèvement 11 en aval de la pompe de circulation 13. Une poche de liquide de substitution 15 est reliée à la canalisation de restitution 12 par l'intermédiaire d'une canalisation de perfusion 16 sur laquelle est disposée une pompe de perfusion 17. Un détecteur de bulles 18 et un organe d'obturation 19 sont placés sur la canalisation de restitution 12, à proximité de la canule. Les trois poches 5, 8, 15 sont suspendues à des balances 20, 21, 22 permettant d'effectuer un bilan des liquides mis en circulation en vue un objectif thérapeutique déterminé.

Pour plus de détails sur cet appareil et sur son fonctionnement on se reportera à la demande de brevet européen n° 0 611 228 et à la demande de brevet européen n° 0 611 227 qui décrivent respectivement un appareil de ce type et un module de traitement de liquide à usage unique particulièrement adapté à cet appareil.

L'appareil qui vient d'être brièvement décrit est destiné au traitement de patients dits "aigus", lesquels, par opposition aux patients dits "chroniques" dont chaque séance de traitement ne dure que quelques heures, peuvent nécessiter des séances de traitement durant plusieurs jours. Il est donc du plus haut intérêt de doter ce type d'appareil de moyens de mesure de pression de liquide ne présentant pas les déficiences mentionnées plus haut, c'est-à-dire n'obligeant pas à remplacer le circuit hydraulique plus qu'il n'est nécessaire au plan thérapeutique.

L'appareil de la figure 1 est muni de quatre dispositifs de mesure de pression de liquide 23, 24, 25, 26, disposés respectivement sur la canalisation de prélèvement 11, en amont et en aval de la pompe de circulation 13, sur la canalisation de restitution 12 et sur la canalisation d'évacuation 9. Ces dispositifs fournissent des informations de pression à une unité de calcul et de commande 27 qui pilote chacun de ces dispositifs à partir des informations fournies et d'un programme de réglage préalablement mis en mémoire. Les fonctions spécifiques de cette unité de commande seront détaillées plus loin.

La figure 2 représente l'un des dispositifs de mesure de pression selon l'invention. Ce dispositif comprend un boîtier cylindrique 30, divisé en deux compartiments 31, 32 par une membrane circulaire souple 33, faite de silicone par exemple. La membrane 33 présente deux zones concentriques jointes par un congé arrondi, une zone périphérique par laquelle la membrane 33 est assujettie au boîtier 30 et une zone centrale qui, lorsque la membrane est au repos, est décalée latéralement par rapport à la zone périphérique. Usuellement, la membrane 33 est montée dans le boîtier de façon à avoir un de débattement maximal compte tenu des pressions moyennes régnant dans la canalisation de liquide (surpression ou dépression par rapport à la pression du gaz dans le second compartiment 32, en général la pression atmosphérique) . Sur la figure 2, le montage de la membrane 33 convient à une canalisation où règne une dépression (cas du dispositif 23). Le premier compartiment 31 du boîtier 30 a une entrée et une sortie connectées à la canalisation où circule le liquide dont la pression est à mesurer. La paroi du boîtier 30 délimitant le second compartiment 32 comprend un orifice central 34 entouré d'un épaulement cylindrique extérieur 35. L'épaulement 35 constitue des moyens de raccord pour coopérer avec une tubulure de raccord 36 complémentaire formant l'extrémité d'une canalisation 37 reliée à un capteur de pression 38 sensible à la pression gazeuse. Ce capteur de pression 38 est constitué, par exemple, par une plaquette semiconductrice déformable. La tubulure 36 comporte une portion d'extrémité ayant un diamètre extérieur inférieur au diamètre intérieur de l'épaulement cylindrique 35 ; un joint torique 39 monté dans une nervure circulaire pratiquée dans la portion d'extrémité de la tubulure ; et un épaulement radial 40 faisant saillie sur la tubulure 36 et destiné à limiter la pénétration de la tubulure 36 dans l'épaulement cylindrique 35 du boîtier 30. La taille du joint torique 39 est choisie pour que, une fois le boîtier 30 emboîté sur la tubulure 36 et l'épaulement cylindrique 35 en butée contre l'épaulement radial 40, le raccordement du compartiment 32 à la tubulure 36 soit étanche.

Conformément à l'invention, le dispositif de mesure de pression comporte des moyens pour positionner automatiquement la membrane 33 dans le boîtier 30 dans une position déterminée. Dans le mode de réalisation représenté, ces moyens comprennent une pompe péristaltique 41 apte à aspirer ou à refouler de l'air par une canalisation 42 reliée à la canalisation 37 par l'intermédiaire d'une vanne 43 permettant de mettre sélectivement en communication la pompe 41 et le second compartiment 32 du boîtier 30. L'extrémité libre de la canalisation 42 est munie d'un filtre 44. Sur la canalisation 42 est disposé un capteur de pression de réglage 45.

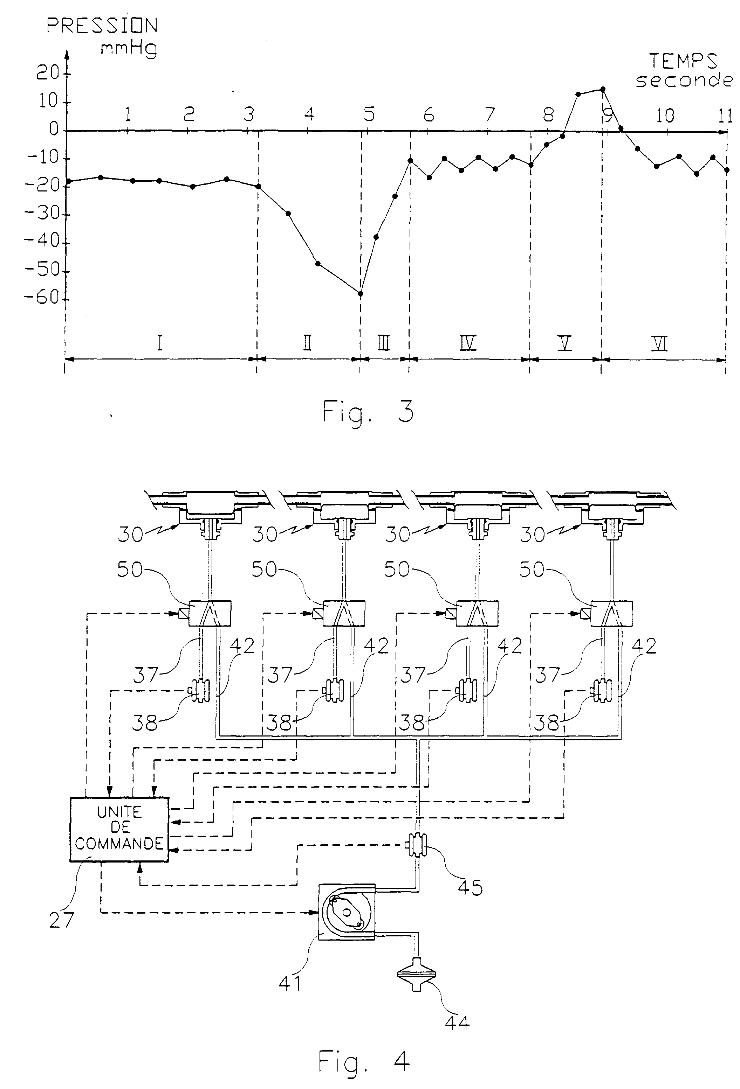

On se reportera à la figure 3 pour suivre l'explication du fonctionnement du dispositif de la figure 2.

En fonctionnement normal, la vanne 43 est fermée. Le capteur de pression 38 émet en permanence un signal représentatif de la pression du liquide circulant dans le premier compartiment 31 du boîtier 30. Après un traitement dans un circuit électronique, le signal est fourni a une unité de commande où il est utilisé essentiellement à des fins de contrôle et de sécurité.

A intervalles de temps réguliers, on procède à une phase de réglage du dispositif, qui consiste à repositionner la membrane 33 dans une position optimale à partir de la position qu'elle occupe alors. Une étape préparatoire à cette phase de réglage consiste à comparer les réponses des deux capteurs de pression 38 et 45 et à ajuster, le cas échéant, la pression dans la canalisation 42 au moyen de la pompe 41, de façon que cette pression soit sensiblement égale à la pression dans le deuxième compartiment 32. De la sorte, lorsque la vanne 43 est ouverte, la membrane 33 n'est soumise à aucune variation de pression, ce qui pourrait être le cas si on ne procédait pas à cette étape d'équilibrage de pression initiale. Après ouverture de la vanne 43, la pompe 41 est mise en rotation dans un sens ou dans l'autre - ici, de façon à retirer de l'air du compartiment 32. Aussi longtemps que la membrane 33 ne vient pas se coller contre la paroi du boîtier 30 délimitant le compartiment 32, la pression mesurée par le capteur 38 reste sensiblement constante (période I). Lorsque la membrane 33 s'est collée sur la paroi, la pression mesurée décroît rapidement (période II). Lorsque la pression atteint une valeur de seuil basse préalablement mise en mémoire dans l'unité de commande 27, le sens de rotation de la pompe 41 est inversé, c'est-à-dire que de l'air est introduit dans le compartiment 32. La pression augmente rapidement (période III) puis atteint un plateau (période IV) correspondant à une position flottante de la membrane 33. A partir du moment où la membrane 33 vient en butée contre la paroi du boîtier 30 délimitant le premier compartiment 31, la pression augmente à nouveau (période V). Quand la pression atteint une valeur de seuil haute préalablement mise en mémoire dans l'unité de commande 27, le sens de rotation de la pompe 41 est à nouveau inversé pour une durée prédéterminée, correspondant à une position optimale de la membrane 33 dans le boîtier 30. Lorsque le débit de la pompe 41 est réglé pour être constant, que la pompe 41 fonctionne en aspiration ou en refoulement, cette durée prédéterminée peut être calculée pour être égale la moitié du temps s'écoulant entre les deux inversions consécutives du sens de rotation de la pompe 41, c'est-à-dire la moitié de la somme des périodes III, IV et V.

Les valeurs de seuil basse et haute mentionnées plus haut sont, de préférence, calculées en terme de variation à partir de la valeur de pression mesurée dans la chambre 32 avant le début de la phase de réglage du dispositif qui vient d'être décrite.

La position de la membrane 33 peut être réglée de différentes façons, dont celle qui vient d'être décrite n'est qu'un exemple. En particulier, dans le procédé décrit ci-dessus, on a considéré qu'au début de chaque phase de réglage la membrane 33 est dans une position flottante correspondant à un plateau de pression. Cette supposition est exacte aussi longtemps que la fréquence des phases de réglage successives est assez élevée pour que, même si l'ensemble boîtier 30/capteur de pression 38 fuit ou si la perméation du gaz au travers de la membrane 33 est importante, la membrane 33 ne vient jamais en butée contre l'une ou l'autre des parois opposées du boîtier entre deux phases de réglage successives.

Une variante du procédé décrit ci-dessus, dans laquelle la position de la membrane 33 dans le boîtier peut être réglée quelle que soit sa position initiale, est exposée dans ce qui suit.

Avant la phase de réglage elle-même, la pompe 41 est mise en service de façon que les pressions des deux côtés de la vanne 43, telles que mesurées par les capteurs de pression 38, 45, soient sensiblement égales, c'est-à-dire la pression dans le second compartiment 32 du boîtier 30 et la pression dans la canalisation 42 reliant la pompe 41 à la vanne 43. Lorsque l'équilibre des pressions est obtenue, la vanne 43 est ouverte.

La pompe 41 est alors mise en service de façon à injecter de l'air dans le second compartiment 32 et la pression qui y règne est mesurée de façon continue par le capteur de pression 45. En fonction de l'évolution de la pression, c'est-à-dire en fonction de la position initiale de la membrane 33, la phase de réglage est effectuée de trois façons différentes :
1 - si la pression reste sensiblement constante (plateau de pression), puis augmente jusqu'à atteindre un seuil de pression relatif prédéterminé (plateau de pression + ΔP), ou si en moins d'une période de temps déterminé T1, la pression atteint un plateau puis augmente jusqu'à un seuil de pression relatif prédéterminé (plateau de pression + ΔP), lorsque le seuil prédéterminé est atteint, le sens de rotation de la pompe 41 est inversé et la rotation de la pompe est commandée pendant une période de temps déterminée T4.
2 - si la pression augmente et atteint, avant qu'une période de temps déterminée T2 se soit écoulée, un seuil de pression supérieur absolu Pmax, le sens de rotation de la pompe 41 est inversé et la rotation de la pompe est commandée jusqu'à que la pression atteigne un seuil de pression relatif prédéterminé (plateau de pression - ΔP). Pendant cette étape, la pression décroît, reste sensiblement constante pendant une période de plateau, puis décroît à nouveau. Lorsque la pression atteint le seuil de pression prédéterminé (plateau de pression - ΔP), le sens de la rotation de la pompe 41 est inversé et la rotation de la pompe est commandée pendant une période de temps T4 déterminée.
3 - si la pression augmente et n'atteint pas, avant qu'une période de temps déterminé T3 se soit écoulée, un plateau de pression ou un seuil de pression supérieur absolu prédéterminé Pmax, le sens de rotation de la pompe 41 est inversé : en fonction de l'évolution de la pression, la phase de réglage est effectuée selon l'une des deux façons suivantes :
   a - si la pression atteint un plateau de pression, la rotation de la pompe 41 est commandée jusqu'à ce que la pression atteigne un seuil de pression relatif prédéterminé (plateau de pression - ΔP) et lorsque le seuil prédéterminé est atteint, le sens de rotation de la pompe 41 est inversé et la rotation de la pompe est commandée pendant une période de temps déterminée T4.
   b - si la pression atteint un seuil de pression inférieur absolu prédéterminé Pmin, le sens de rotation de la pompe 41 est inversé et la rotation de la pompe 41 est commandée jusqu'à ce que la pression atteigne un seuil de pression relatif prédéterminé (plateau de pression + ΔP). Durant cette étape la pression augmente, reste sensiblement constante pendant une période de plateau, puis augmente à nouveau. Lorsque le seuil de pression relatif prédéterminé est atteint, le sens de rotation de la pompe est inversé et la rotation de la pompe est commandée pendant une période de temps déterminée T4.

Selon ce procédé de réglage, quelque soit la valeur de la pression initiale, c'est-à-dire quelque soit la position de la membrane au début de la phase de réglage, un plateau de pression est d'abord recherché, puis la rotation de la pompe 41 est commandée jusqu'à ce que la pression atteigne un seuil de pression relatif prédéterminé (plateau de pression + ΔP ou plateau de pression - ΔP). Lorsque ce seuil est atteint, le sens de rotation de la pompe 41 est inversé et la rotation de la pompe est commandée pendant une période de temps déterminée T4.

Lors de la mise en oeuvre du procédé qui vient d'être décrit, la pompe est toujours mise en service initialement de façon à injecter de l'air dans le boîtier. On pourrait naturellement prévoir de la mettre en service de façon qu'elle aspire de l'air hors du boîtier.

Selon une variante, la pompe peut être entraînée par un moteur pas à pas. Dans ce cas au lieu que dans les étapes de procédé décrites précédemment la rotation de la pompe soit commandée pendant une période de temps déterminé, elle peut être commandée pendant un nombre de pas déterminés.

T4 peut être déterminée empiriquement en prenant en considération la forme et le volume interne du boîtier et les conditions de fonctionnement du dispositif de mesure de pression. Dans un mode de réalisation de l'invention, les périodes de temps T1, T2, T3 sont choisies égales.

Une fois le réglage du dispositif achevé, la vanne 44 est fermée à nouveau et le boîtier 30 est isolé de la pompe 41.

Toutes les étapes du procédé de réglage du dispositif selon l'invention qui on été décrites sont préférentiellement commandées et séquencées par l'unité de calcul et de commande 27 qui comprend un microprocesseur et la capacité de mémoire nécessaires pour stocker le programme de déroulement du réglage et les valeurs de seuil hautes et basses utilisées.

La figure 4 représente un agencement particulier des dispositifs de mesure de pression de l'appareil représenté sur la figure 1. Dans cet agencement les quatre dispositifs de mesure de pression 23, 24, 25, 26 ont en commun la pompe 41 et le capteur de pression de réglage 45. A la différence du dispositif de la figure 2, chacun de ces dispositifs comporte une vanne trois voies 50 permettant de mettre sélectivement en communication le second compartiment 32 du boîtier 30 avec le capteur de mesure 38 ou le second compartiment 32 du boîtier 30 avec la pompe 41 et le capteur de réglage 45. Grâce à ce montage, il est possible de vérifier le fonctionnement du capteur de mesure 38 en comparant la valeur de pression mesurée par le capteur de mesure 38 après le repositionnement de la membrane 33 avec la valeur de pression mesurée par le capteur de réglage 45 pendant les périodes I ou IV.

Le fonctionnement de ces dispositifs n'est pas différent de celui qui vient d'être expliqué par référence aux figures 3 et 4. Les phases de réglages des différents dispositifs sont commandées séquentiellement, l'une après l'autre, selon un programme mis en mémoire dans l'unité de commande 27.

L'invention est susceptible de variantes et de modifications. En particulier, le champ de l'invention s'étend aussi à une version simplifiée du dispositif représenté sur la figure 2, où la tubulure de raccordement 36 serait reliée directement à la pompe 41, sans possibilité d'isoler la pompe du boîtier 30 par une vanne.

## Revendications

1. Dispositif de mesure de la pression d'un liquide comportant:
- un capteur de pression (38) sensible à une variation de pression gazeuse ;
- un boîtier (30) divisé en deux compartiments (31, 32) par une membrane souple (33) ayant deux faces opposées tournées respectivement vers chacun des deux compartiments (31, 32), un premier compartiment (31) ayant au moins un orifice pour l'introduction du liquide et un second compartiment (32) étant connectable de façon étanche au capteur de pression (38) ; et
- des moyens de pompage (41) pour faire varier la quantité de gaz dans le deuxième compartiment (32),
- des moyens de commande (27) pour piloter les moyens de pompage (41) en fonction des variations de pression induites dans le second compartiment (32) par les moyens de pompage (41) de façon à ajuster la position de la membrane (33) entre une paroi du boîtier (30) délimitant le premier compartiment (31) et une paroi du boîtier (30) délimitant le deuxième compartiment (32).

2. Dispositif selon la revendication 1, caractérisé en ce que le deuxième compartiment (32) est selectivement connectable au capteur de pression (38) ou aux moyens de pompage (41).

3. Dispositif selon une des revendications 1 et 2, caractérisé en ce que les moyens de pompage (41) sont connectables au deuxième compartiment (32) du boîtier (30) par une canalisation (42) obturable par une vanne (43).

4. Dispositif selon la revendication 3, caractérisé en ce qu'il comporte un second capteur de pression (45) connecté à la canalisation (42) reliant la vanne (43) aux moyens de pompage (41).

5. Dispositif selon la revendication 4, caractérisé en ce qu'il comporte des moyens (27) pour comparer les informations mesurées par le premier et le second capteur de pression (38, 45) et pour piloter les moyens de pompage (41) de façon que les pressions dans la canalisation (37, 42) de part et d'autre de la vanne (43) soient sensiblement égales.

6. Appareil de traitement de sang par circulation extracorporelle comprenant un pluralité de canalisations (6, 9, 11, 12) pour la circulation d'au moins un liquide et au moins deux dispositifs (30, 38, 41) selon une des revendications 1 à 4 pour mesurer une pression dans au moins une canalisation (11).

7. Appareil selon la revendication 6, caractérisé en ce qu'il comporte
- un dispositif de traitement de sang (1) connectable à un patient par une canalisation de prélèvement de sang (11) et par une canalisation de restitution de sang (12) ;
- au moins un dispositif de mesure de pression (30, 38, 41) pour mesurer la pression du sang dans la canalisation de prélèvement (11); et
- au moins un dispositif de mesure de pression (30, 38, 41) pour mesurer la pression du sang dans la canalisation de restitution (12).

8. Appareil selon la revendication 7, caractérisé en ce qu'il comporte deux dispositifs de mesure de pression (30, 38, 41) pour mesurer la pression du sang dans la canalisation de prélèvement (11), respectivement en amont et en aval d'une pompe de circulation (13) disposée sur la canalisation de prélèvement (11).

9. Appareil selon une des revendications 7 et 8, caractérisé en ce que
- le dispositif de traitement du sang est un échangeur à membrane semi-perméable (1) ayant une première chambre (3) à laquelle sont connectées la canalisation de prélèvement (11) et la canalisation de restitution (12) de sang, et une seconde chambre (2) à laquelle sont connectées une canalisation d'alimentation (6) en liquide de traitement et une canalisation d'évacuation (9) de liquide usé, et
- la canalisation d'évacuation (9) de liquide usé est munie d'un dispositif de mesure de pression (30, 38, 41).

10. Appareil selon une des revendications 6 à 9, caractérisé en ce qu'il comporte des moyens de pompage (41) uniques pour faire varier une quantité de gaz, connectables sélectivement au deuxième compartiment (32) de chaque boîtier (30) de mesure de pression.

11. Appareil selon une des revendications 6 à 10, caractérisé en ce que les moyens pour faire varier une quantité de gaz comprennent une pompe (41) apte à aspirer et à refouler alternativement par deux orifices, un premier orifice étant relié à l'atmosphère par un filtre (44) et un second orifice étant connectable au deuxième compartiment (32) du boîtier (30).

12. Procédé de réglage d'un dispositif de mesure de la pression d'un liquide comportant :
- un capteur de pression (38) sensible à une variation de pression gazeuse et
- un boîtier (30) divisé en deux compartiments (31, 32) par une membrane souple (33) ayant deux faces opposées tournées respectivement vers chacun des deux compartiments, un premier compartiment (31) ayant un orifice pour l'introduction de liquide et un second compartiment (32) étant connectable de façon étanche au capteur de pression (38),
caractérisé en ce qu'il comporte les étapes de :
- introduire dans le premier compartiment (31) le liquide dont la pression est à mesurer ;
- faire varier la quantité de gaz dans le second compartiment (32) au moins une fois dans un sens et au moins une fois dans le sens opposé jusqu'à ce que que le second compartiment (32) contiennent une quantité de gaz correspondant à une position déterminée de la membrane (33) dans le boîtier (30).

13. Procédé selon la revendication 12, caractérisé en ce que l'étape de faire varier la quantité de gaz dans le second compartiment (32) comprend les étapes de :
- faire varier dans un sens la quantité de gaz dans le second compartiment (32) jusqu'à ce que la pression du gaz atteigne une valeur de seuil déterminée ;
- régler la position de la membrane (33) dans le boîtier (30) en faisant varier en sens contraire la quantité de gaz dans le second compartiment (32).

14. Procédé selon la revendication 13, caractérisé en ce que l'étape de faire varier la quantité de gaz dans le second compartiment (32) comprend, après l'étape de faire varier dans un sens la quantité de gaz, l'étape additionnelle de faire varier en sens contraire la quantité de gaz jusqu'à ce que la pression du gaz dans le second compartiment (32) atteigne une seconde valeur de seuil déterminée.

15. Procédé selon la revendication 12, caractérisé en ce que l'étape de faire varier la quantité de gaz dans le second compartiment (32) comprend les étapes de :
- faire varier la quantité de gaz dans le second compartiment (32) jusqu'à ce que la pression du gaz dans le second compartiment (32) atteigne une valeur de plateau correspondant à une position de flottement de la membrane (33), puis évolue dans un seul sens et atteigne une valeur de seuil déterminée ;
- régler la position de la membrane (33) dans le boîtier (30) en faisant varier en sens contraire la quantité de gaz dans le second compartiment (32).

16. Procédé selon la revendication 15, caractérisé en ce que l'étape de faire varier la quantité de gaz dans le second compartiment (32) jusqu'à ce que la pression du gaz dans le second compartiment (32) atteigne une valeur de plateau consiste à faire varier dans un seul sens la quantité de gaz dans le second compartiment (32).

17. Procédé selon la revendication 15, caractérisé en ce que l'étape de faire varier la quantité de gaz dans le second compartiment (32) jusqu'à ce que la pression du gaz dans le second compartiment (32) atteigne une valeur de plateau comprend les étapes de :
- faire varier dans un sens la quantité de gaz dans le second compartiment (32) jusqu'à ce que la pression atteigne un seuil de pression absolue prédéterminé, si ce seuil de pression absolue est atteint en moins d'une période de temps prédéterminée ;
- faire varier en sens contraire la quantité de gaz dans le second compartiment (32).

18. Procédé selon la revendication 15, caractérisé en ce que l'étape de faire varier la quantité de gaz dans le second compartiment (32) jusqu'à ce que la pression du gaz dans le second compartiment (32) atteigne une valeur de plateau comprend les étapes de :
- faire varier dans un sens la quantité de gaz dans le second compartiment (32) pendant une période de temps prédéterminée ;
- faire varier en sens contraire la quantité de gaz dans le second compartiment (32).

19. Procédé selon la revendication 15, caractérisé en ce que l'étape de faire varier la quantité de gaz dans le second compartiment (32) jusqu'à ce que la pression du gaz dans le second compartiment (32) atteigne une valeur de plateau comprend les étapes de
- faire varier dans un sens la quantité de gaz dans le second compartiment (32) pendant une période de temps prédéterminée ;
- faire varier en sens inverse la quantité de gaz dans le second compartiment (32) jusqu'à ce que la pression atteigne un seuil de pression absolue prédéterminé;
- faire varier en sens contraire la quantité de gaz dans le second compartiment (32).

20. Procédé selon une des revendications 13 à 19, caractérisé en ce que l'étape de régler la position de la membrane (33) dans le boîtier (30) consiste à introduire ou à extraire du gaz dans/du second compartiment (32) pendant une durée déterminée.

21. Procédé selon les revendications 14 et 20, caractérisé en ce qu'il comporte en outre l'étape de calculer la durée pendant laquelle du gaz est introduit ou extrait du second compartiment (32) à partir de la durée s'écoulant entre la mesure de la pression à la première valeur de seuil et à la seconde valeur de seuil.

22. Procédé selon une des revendications 12 à 21, caractérisé en ce que l'étape de faire varier la quantité de gaz dans le second compartiment (32) successivement dans des sens opposés consiste à introduire ou à extraire du gaz du second compartiment (32) au moyen d'une pompe (41) fonctionnant à un débit sensiblement constant.

23. Procédé selon une des revendications 13 à 18, caractérisé en ce que chaque valeur de pression de seuil utilisée comme référence pour déclencher l'étape de réglage de la position de la membrane (33) est calculée en terme de variation de pression relative à partir de la valeur de pression mesurée dans le second compartiment (32) du boîtier (30) avant l'étape d'ajuster la quantité de gaz dans le second compartiment (32).

## Claims

1. Device for measuring the pressure of a liquid, comprising:
- a pressure sensor (38) which is sensitive to a variation in gas pressure ;
- a casing (30) divided into two compartments (31, 32) by a flexible membrane (33) having two opposite sides respectively facing each of the two compartments (31, 32), a first compartment (31) having at least one orifice for the introduction of the liquid and a second compartment (32) being connectable in a leaktight manner to the pressure sensor (38) ;
- pumping means (41) for varying the quantity of gas in the second compartment (32) ;
- control means (27) for driving the pumping means (41) as a function of the variations of pressure generated in the second compartment (32) by the pumping means (41) so as to adjust the position of the membrane (33) between a wall of the casing (30) bounding the first compartment (31) and a wall of the casing (30) bounding the second compartment (32).

2. Device according to Claim 1, characterized in that the second compartment (32) is selectively connectable to the pressure sensor (38) or to the pumping means (41).

3. Device according to one of Claims 1 and 2, characterized in that the pumping means (41) are connectable to the second compartment (32) of the casing (30) by a pipe (42) which can be closed by a valve (43).

4. Device according to Claim 3, characterized in that it comprises a second pressure sensor (45) connected to the pipe (42) joining the valve (43) to the pumping means (41).

5. Device according to Claim 4, characterized in that it comprises means (27) for comparing the data measured by the first and second pressure sensors (38, 45) and for driving the pumping means (41) so that the pressures in the pipes (37, 42) on either side of the valve (43) are substantially equal.

6. Apparatus for treating blood by extracorporeal circulation, comprising a plurality of pipes (6, 9, 11, 12) for circulation of at least one liquid and at least two devices (30, 38, 41) according to one of Claims 1 to 5 for measuring a pressure in at least one pipe (11).

7. Apparatus according to Claim 6, characterized in that it comprises :
- a blood treatment device (1) connectable to a patient by a blood withdrawal pipe (11) and by a blood return pipe (12) ;
- at least one pressure measurement device (30, 38, 41) for measuring the pressure of the blood in the withdrawal pipe (11); and
- at least one pressure measurement device (30, 38, 41) for measuring the pressure of the blood in the return pipe (12).

8. Apparatus according to Claim 7, characterized in that it comprises two pressure measurement devices (30, 38, 41) for measuring the pressure of the blood in the withdrawal pipe (11), respectively upstream and downstream of a circulation pump (13) arranged on the withdrawal pipe (11).

9. Apparatus according to one of Claims 7 and 8, characterized in that :
- the blood treatment device is a semi-permeable membrane exchanger (1) having a first chamber (3) to which the blood withdrawal pipe (11) and the blood return pipe (12) are connected, and a second chamber (2) to which a treatment liquid supply pipe (6) and a spent liquid discharge pipe (9) are connected, and
- the spent liquid discharge pipe (9) is fitted with a pressure measurement device (30, 38, 41).

10. Apparatus according to one of Claims 6 to 9, characterized in that it comprises single pumping means (41) for varying a quantity of gas, which are connectable selectively to the second compartment (32) of each pressure measurement casing (30).

11. Apparatus according to one of Claims 6 to 10, characterized in that the means for varying a quantity of gas comprise a pump (41) capable of suction and delivery alternately via two orifices, a first orifice being connected to the atmosphere via a filter (44) and a second orifice being connectable to the second compartment (32) of the casing (30).

12. Method for regulating a device for measuring the pressure of a liquid, comprising:
- a pressure sensor (38) which is sensitive to a variation in gas pressure, and
- a casing (30) divided into two compartments (31, 32) by a flexible membrane (33) having two opposite sides respectively facing each of the two compartments (31, 32), a first compartment (31) having an orifice for the introduction of liquid and a second compartment (32) being connectable in a leaktight manner to the pressure sensor (38),
characterized in that it comprises the steps of:
- introducing the liquid whose pressure is to be measured into the first compartment (31) ;
- varying the quantity of gas in the second compartment (32) at least in one sense and at least in the opposite sense so that the second compartment (32) contains a determined quantity of gas corresponding to a determined position of the membrane (33) in the casing (30).

13. Method according to Claim 12, characterized in that the step of varying the quantity of gas in the second compartment (32) comprises the steps of :
- varying the quantity of gas in the second compartment (32) in one sense until the pressure of the gas reaches a determined threshold value;
- adjusting the position of the membrane (33) in the casing (30) by varying the quantity of gas in the second compartment (32) in the opposite sense.

14. Method according to Claim 13, characterized in that the step of varying the quantity of gas in the second compartment (32) comprises, after the step of varying the quantity of gas in one sense, the additional step of varying the quantity of gas in the opposite sense until the pressure of the gas in the second compartment (32) reaches a second determined threshold value.

15. Method according to Claim 12, characterized in that the step of varying the quantity of gas in the second compartment (32) comprises the steps of :
- varying the quantity of gas in the second compartment (32) until the pressure of the gas in the second compartment (32) reaches a plateau pressure corresponding to a floating position of the membrane (33), and then evolves in a single sense and reaches a determined threshold value ;
- adjusting the position of the membrane (33) in the casing (30) by varying the quantity of gas in the second compartment (32) in the opposite sense.

16. Method according to Claim 15, characterized in that the step of varying the quantity of gas in the second compartment (32) until the pressure of the gas in the second compartment (32) reaches a plateau pressure consists in varying in a single sense the quantity of gas in the second compartment (32).

17. Method according to Claim 15, characterized in that the step of varying the quantity of gas in the second compartment (32) until the pressure of the gas in the second compartment (32) reaches a plateau pressure comprises the steps of :
- varying the quantity of gas in the second compartment (32) in one sense until the pressure of the gas reaches a determined absolute threshold value, whenever this absolute threshold value is reached in less than a determined period of time ;
- varying the quantity of gas in the second compartment (32) in the opposite sense.

18. Method according to Claim 15, characterized in that the step of varying the quantity of gas in the second compartment (32) until the pressure of the gas in the second compartment (32) reaches a plateau pressure comprises the steps of :
- varying the quantity of gas in the second compartment (32) in one sense for a determined period of time ;
- varying the quantity of gas in the second compartment (32) in the opposite sense.

19. Method according to Claim 15, characterized in that the step of varying the quantity of gas in the second compartment (32) until the pressure of the gas in the second compartment (32) reaches a plateau pressure comprises the steps of :
- varying the quantity of gas in the second compartment (32) in one sense for a determined period of time ;
- varying the quantity of gas in the second compartment (32) in the opposite sense until the pressure of the gas reaches a determined absolute threshold value;
- varying the quantity of gas in the second compartment (32) in the opposite sense.

20. Method according to one of Claims 13 to 19, characterized in that the step of adjusting the position of the membrane (33) in the casing (30), consists in introducing or extracting gas into or from the second compartment (32) for a determined period of time.

21. Method according to Claims 14 and 20, characterized in that it further comprises the step of calculating the period of time during which gas is introduced into or extracted from the second compartment (32) on the basis of the period of time which has elapsed between the pressure measurement at the first threshold value and that at the second threshold value.

22. Method according to one of Claims 12 to 21, characterized in that the step of varying the quantity of gas in the second compartment (32) successively in one sense and in the opposite sense consists in introducing or extracting gas into or from the second compartment (32) by means of a pump (41) operating at a substantially constant flow rate.

23. Method according to one of Claims 13 to 18, characterized in that each threshold pressure value used as a reference for starting the step of adjusting the position of the membrane (33) in the casing (30) is calculated in terms of pressure variation from a pressure value measured in the second compartment (32) of the casing (30) before the step of adjusting the quantity of gas in the second compartment (32).

## Patentansprüche

1. Vorrichtung zur Messung des Drucks einer Flüssigkeit mit:
- einem auf eine Gasdruckänderung empfindlichen Druckaufnehmer (38);
- einem Behältnis (30), das in zwei Teilräume (31, 32) unterteilt wird durch eine biegsame Membran (33), die zwei gegenüberliegende Seiten aufweist, die jeweils jedem der beiden Teilräume (31, 32) zugewandt sind, wobei ein erster Teilraum (31) zumindest eine Öffnung für die Einleitung von Flüssigkeit aufweist und ein zweiter Teilraum (32) auf luftdichte Weise mit dem Druckaufnehmer (38) verbindbar ist; und
- Pumpmitteln (41), um die Gasmenge in dem zweiten Teilraum (32) zu verändern,
- Steuerungsmitteln (27) zur Ansteuerung der Pumpmittel (41) in Abhängigkeit von den in dem zweiten Teilraum (32) durch die Pumpmittel (41) induzierten Druckänderungen, um so die Position der Membran (33) zwischen einer den ersten Teilraum (31) begrenzenden Wand des Behältnisses (30) und einer den zweiten Teilraum (32) begrenzenden Wand des Behältnisses (30) einzustellen.

2. Vorrichtung nach Anspruch 1, dadurch gekennzeichnet, daß der zweite Teilraum (32) wahlweise mit dem Druckaufnehmer (38) oder den Pumpmitteln (41) verbindbar ist.

3. Vorrichtung nach einem der Ansprüche 1 und 2, dadurch gekennzeichnet, daß die Pumpmittel (41) mit dem zweiten Teilraum (32) des Behältnisses (30) über eine durch ein Ventil (43) verschließbare Leitung (42) verbindbar sind.

4. Vorrichtung nach Anspruch 3, dadurch gekennzeichnet, daß sie einen zweiten Druckaufnehmer (45) umfaßt, der an die Leitung (42) angeschlossen ist, die das Ventil (43) mit den Pumpmitteln (41) verbindet.

5. Vorrichtung nach Anspruch 4, dadurch gekennzeichnet, daß sie Mittel (27) umfaßt, um die durch den ersten und den zweiten Druckaufnehmer (38, 45) gemessenen Informationen zu vergleichen und um die Pumpmittel (41) so anzusteuern, daß die Drücke in der Leitung (37, 42) auf beiden Seiten des Ventils (43) im wesentlichen gleich sind.

6. Vorrichtung zur Behandlung von Blut mit extrakorporalem Kreislauf mit einer Mehrzahl von Leitungen (6, 9, 11, 12) zum Umwälzen zumindest einer Flüssigkeit und mit zumindest zwei Vorrichtungen (30, 38, 41) gemäß einem der Ansprüche 1 bis 4 zum Messen eines Druckes in zumindest einer Leitung (11).

7. Vorrichtung nach Anspruch 6, dadurch gekennzeichnet, daß sie folgendes umfaßt:
- eine Vorrichtung zur Behandlung von Blut (1), die durch eine Blutentnahmeleitung (11) und durch eine Blutrückführungsleitung (12) mit einem Patienten verbindbar ist;
- zumindest eine Druckmeßvorrichtung (30, 38, 41) zum Messen des Blutdrucks in der Entnahmeleitung (11); und
- zumindest eine Druckmeßvorrichtung (30, 38, 41) zum Messen des Blutdrucks in der Rückführungsleitung (12).

8. Vorrichtung nach Anspruch 7, dadurch gekennzeichnet, daß sie stromauf beziehungsweise stromab von einer in der Entnahmeleitung (11) angeordneten Umwälzpumpe (13) zwei Druckmeßvorrichtungen (30, 38, 41) zum Messen des Blutdrucks in der Entnahmeleitung (11) umfaßt.

9. Vorrichtung nach einem der Ansprüche 7 und 8, dadurch gekennzeichnet, daß
- die Blutbehandlungsvorrichtung ein Austauscher mit halbdurchlässiger Membran (1) ist, der eine erste Kammer (3), an die die Blutentnahmeleitung (11) und die Blutrückführungsleitung (12) angeschlossen sind, und eine zweite Kammer (2), an die eine Versorgungsleitung (6) für Behandlungsflüssigkeit und eine Abflußleitung (9) für verbrauchte Flüssigkeit angeschlossen sind, besitzt und
- die Abflußleitung (9) für verbrauchte Flüssigkeit mit einer Druckmeßvorrichtung (30, 38, 41) versehen ist.

10. Vorrichtung nach einem der Ansprüche 6 bis 9, dadurch gekennzeichnet, daß sie ein einziges Pumpmittel (41) zum Verändern einer Gasmenge umfaßt, das wahlweise an den zweiten Teilraum (32) jedes Druckmeßbehältnisses (30) anschließbar ist.

11. Vorrichtung nach einem der Ansprüche 6 bis 10, dadurch gekennzeichnet, daß die Mittel zum Verändern einer Gasmenge eine Pumpe (41) umfassen, die geeignet ist, abwechselnd durch zwei Öffnungen anzusaugen und auszustoßen, wobei eine erste Öffnung über ein Filter (44) mit der Atmosphäre verbunden ist und eine zweite Öffnung mit dem zweiten Teilraum (32) des Behältnisses (30) verbindbar ist.

12. Verfahren zur Einstellung einer Vorrichtung zur Messung des Drucks einer Flüssigkeit mit:
- einem auf eine Gasdruckänderung empfindlichen Druckaufnehmer (38);
- einem Behältnis (30), das in zwei Teilräume (31, 32) unterteilt wird, durch eine biegsame Membran (33), die zwei gegenüberliegende Seiten aufweist, die jeweils jedem der beiden Teilräume zugewandt sind, wobei ein erster Teilraum (31) eine Öffnung für die Einleitung von Flüssigkeit aufweist und ein zweiter Teilraum (32) auf luftdichte Weise mit dem Druckaufnehmer (38) verbindbar ist,
dadurch gekennzeichnet, daß es die folgenden Schritte umfaßt:
- Einleiten der Flüssigkeit, deren Druck zu messen ist, in den ersten Teilraum (31);
- Verändern der Gasmenge in dem zweiten Teilraum (32), zumindest einmal in eine Richtung und zumindest einmal in die entgegengesetzte Richtung, bis der zweite Teilraum (32) eine Gasmenge enthält, die einer bestimmten Position der Membran (33) in dem Behältnis (30) entspricht.

13. Verfahren nach Anspruch 12, dadurch gekennzeichnet, daß der Schritt des Veränderns der Gasmenge in dem zweiten Teilraum (32) die folgenden Schritte umfaßt:
- Verändern der Gasmenge in dem zweiten Teilraum (32) in eine Richtung, bis der Gasdruck einen bestimmten Grenzwert erreicht;
- Einstellen der Position der Membran (33) in dem Behältnis (30), indem die Gasmenge in dem zweiten Teilraum (32) in entgegengesetzter Richtung verändert wird.

14. Verfahren nach Anspruch 13, dadurch gekennzeichnet, daß der Schritt des Veränderns der Gasmenge in dem zweiten Teilraum (32) nach dem Schritt des Verändern der Gasmenge in eine Richtung den zusätzlichen Schritt des Veränderns der Gasmenge in entgegengesetzter Richtung umfaßt, bis der Gasdruck in dem zweiten Teilraum (32) einen zweiten festgesetzten Grenzwert erreicht.

15. Verfahren nach Anspruch 12, dadurch gekennzeichnet, daß der Schritt des Veränderns der Gasmenge in dem zweiten Teilraum (32) die folgenden Schritte umfaßt:
- Verändern der Gasmenge in dem zweiten Teilraum (32) bis der Gasdruck in dem zweiten Teilraum (32) einen Plateauwert erreicht, der einer schwimmenden Position der Membran (33) entspricht, dann in eine einzige Richtung fortschreitet und einen bestimmten Grenzwert erreicht;
- Verändern die Gasmenge in dem zweiten Teilraum (32) in entgegengesetzter Richtung, um der Position der Membran (33) in dem Behältnis (30) einzustellen.

16. Verfahren nach Anspruch 15, dadurch gekennzeichnet, daß der Schritt des Veränderns der Gasmenge in dem zweiten Teilraum (32) bis der Gasdruck in dem zweiten Teilraum (32) einen Plateauwert erreicht, darin besteht, die Gasmenge in dem zweiten Teilraum (32) in eine einzige Richtung zu verändern.

17. Verfahren nach Anspruch 15, dadurch gekennzeichnet, daß der Schritt des Veränderns der Gasmenge in dem zweiten Teilraum (32) bis der Gasdruck in dem zweiten Teilraum (32) einen Plateauwert erreicht, die folgenden Schritte umfaßt:
- Verändern der Gasmenge in dem zweiten Teilraum (32) in eine Richtung, bis der Druck einen vorbestimmten absoluten Druckgrenzwert erreicht, falls dieser absolute Druckgrenzwert in weniger als einer vorbestimmten Zeitspanne erreicht wird;
- Verändern der Gasmenge in dem zweiten Teilraum (32) in entgegengesetzter Richtung.

18. Verfahren nach Anspruch 15, dadurch gekennzeichnet, daß der Schritt des Veränderns der Gasmenge in dem zweiten Teilraum (32) bis der Gasdruck in dem zweiten Teilraum (32) einen Plateauwert erreicht, die folgenden Schritte umfaßt:
- Verändern der Gasmenge in dem zweiten Teilraum (32) in eine Richtung während einer vorbestimmten Zeitspanne;
- Verändern der Gasmenge in dem zweiten Teilraum (32) in entgegengesetzter Richtung.

19. Verfahren nach Anspruch 15, dadurch gekennzeichnet, daß der Schritt des Veränderns der Gasmenge in dem zweiten Teilraum (32) bis der Gasdruck in dem zweiten Teilraum (32) einen Plateauwert erreicht, die folgenden Schritte umfaßt:
- Verändern der Gasmenge in dem zweiten Teilraum (32) in eine Richtung während einer vorbestimmten Zeitspanne;
- Verändern der Gasmenge in dem zweiten Teilraum (32) in entgegengesetzter Richtung, bis der Druck einen vorbestimmten absoluten Druckgrenzwert erreicht;
- Verändern der Gasmenge in dem zweiten Teilraum (32) in entgegengesetzte Richtung.

20. Verfahren nach einem der Ansprüche 13 bis 19, dadurch gekennzeichnet, daß der Schritt des Einstellens der Position der Membran (33) in dem Behältnis (30) darin besteht, Gas in den zweiten Teilraum (32) während einer bestimmten Dauer einzuleiten oder daraus zu entfernen.

21. Verfahren nach den Ansprüchen 14 und 20, dadurch gekennzeichnet, daß es außerdem den Schritt umfaßt, die Dauer während der Gas eingeleitet oder aus dem zweiten Teilraum (32) entfernt wird, auf der Basis der Dauer zu berechnen, die zwischen der Messung des Drucks beim ersten Grenzwert und dem zweiten Grenzwert verstreicht.

22. Verfahren nach einem der Ansprüche 12 bis 21, dadurch gekennzeichnet, daß der Schritt des aufeinanderfolgenden Veränderns der Gasmenge in dem zweiten Teilraum (32) in entgegengesetzte Richtungen darin besteht, Gas mittels einer Pumpe (41), die mit im wesentlichen konstantem Durchsatz arbeitet, einzuleiten oder aus dem zweiten Teilraum (32) zu entfernen.

23. Verfahren nach einem der Ansprüche 13 bis 18, dadurch gekennzeichnet, daß jeder Druckgrenzwert, der als Bezugswert zum Auslösen des Schritts des Einstellens der Position der Membran (33) verwendet wird, auf der Basis des vor dem Schritt des Einstellens der Gasmenge in dem zweiten Teilraum (32) gemessenen Drucks in dem zweiten Teilraum (32) des Behältnisses (30) in Form einer relativen Druckänderung berechnet wird.
